# EUROPEAN PATENT SPECIFICATION

(11) **EP 0 387 875 B1**
(45) Date of publication and mention of the grant of the patent: **11.10.1995**
(21) Application number: 90104919.7
(22) Date of filing: 15.03.1990
(51) Int. Cl.: C12Q 1/48, G01N 33/573, G01N 33/574, G01N 33/80, G01N 33/66, G01N 33/543

(54) **Method for determination of activities of glycosyltransferases and its use**
Verfahren zur Bestimmung von Aktivitäten von Glycosyltransferasen und dessen Anwendung
Méthode de détermination d'activités de glycosyltransférases et son utilisation

(30) Priority: 15.03.1989 JP 64457/89
(43) Date of publication of application: 19.09.1990
(73) Proprietor: Otsuka Pharmaceutical Company Limited, Chiyoda-ku Tokyo-to (JP)
(72) Inventor: Yazawa, Shin, RC2-106, Higashimaebashijyutaku, Maebashi-shi, Gunma-ken (JP)
(74) Representative: Hansen, Bernd, Dr. Dipl.-Chem.

(56) References cited:
- EP-A- 0 272 603
- WO-A-80/02296

## Description

### FIELD OF THE INVENTION

The present invention relates to a novel method for the determination of enzymatic activities. More particularly, it relates to a method for the quantitative determination of activities of glycosyltransferases which are concerned in biosynthesis of sugar chains of oligosaccharides, so-called glycoconjugates such as glycoproteins and glycolipids and the like. The present invention also relates to the use of the method in medical and clinical tests such as blood grouping, diagnosis of cancer and the like.

### BACKGROUND OF THE INVENTION

Glycosyltransferases are widely present in the biological world from higher animals to microorganisms and, since they are directly responsible for biosynthesis of various sugar chain antigens, their biological role has been noted. For example, recently, it have been known that changes of sugar chain structures of glycoconjugate chains are caused by various diseases, particularly, cancerization. Examples thereof include increase in fucosylation of α1→3 in neolacto series (type 2 sugar chain) with cancerization, and extension of non-branching sugar chains [J.B.C., 259 (16), 10511 - 10517, (1984); J.B.C., 259 (7), 4672 - 4680, (1984); Cancer Res., 46, 2619 - 2626, (1986); J.B.C., 259 (7), 4681 - 4685, (1984); Cancer Res., 48, 475 - 482, (1988); and Cancer Res., 46, 5985 - 5992, (1986)], as well as increase of sialylation in not only neolacto series but also in other sugar chains with cancerization [Cancer Res., 46, 2619 - 2626, (1986); Cancer Res., 48, 2214 - 2220, (1988); and Cancer Res., 45, 2405 - 2414, (1985)]. It is considered that glycosyltransferases are associated with these changes in sugar chain structures. Furthermore, it is also very likely that changes of glycosyltransferases concerned in synthesis of respective sugar chain structures are not only quantitative but qualitative. Therefore, it has been proposed that the determination of glycosyltransferase activities can be used as means for the diagnosis of cancer.

In general, glycosyltransferase activities are determined by combining activated sugar donors, such as sugar nucleotides, with sugar acceptors accepting the sugars from the donors as substrates. However, there are still many technical problems in determination of activities of glycosyltransferases and it has been necessary to develop a simple and more precise determination system because of the importance of glycosyltransferase activities in medical and biological studies as well as practical studies in clinical field.

In the methods for the determination of glycosyltransferase activities which have hitherto been developed, usually, there is employed a method which comprises reacting a ³H- or ¹⁴C-labeled sugar nucleotide with a sugar acceptor in a solution state, separating the resulting enzymatic reaction product by means of, for example, electrophoresis, various chromatography and the like, and determining the radioactivity of the reaction product. However, in these methods, separation of the reaction product is complicated, making it impossible to determine a lot of samples at one time. Further, a simple method has been reported for the determination of glycosyltransferases involved in synthesis of human blood group substances. This method comprises carrying out an enzymatic reaction with red blood cells and detecting blood type conversion of the red blood cells with a blood type specific antibody to determine the enzymatic activities. However, this is a qualitative determination method, rather than a quantitative determination method.

Recent improvements of these determination methods include a method comprising reacting a ¹⁴C-labeled sugar nucleotide with an insoluble oligosaccharide SYNSORB® (manufactured by CHEMBIOMED) and determining an incorporated radioisotope with a scintillation counter [M.J. Elices and I.J. Goldstein, Arch. Biochem. Biophys., 254, 329 - 341, (1987)]. Another proposed method comprises reacting a non-labeled sugar nucleotide with a sugar acceptor and determining the enzymatic activities according to a conventional method by utilizing a substance specifically binding to only the acceptor wherein the sugar has been transferred (Japanese Patent Kokai No. 63-152998).

In the former method, the problem of complicated separation of the reaction product is solved. However, there is no solution to the increase in the background value due to non-specific incorporation and the like, which is also a disadvantage in a conventional method. Furthermore, in the assay by sugar donor uptake, the binding form of the sugar (i.e., α2→3) can not be specified. And, in practice, because of low sensitivity of the β-rays of ¹⁴C, ³H and the like, in general, it is very difficult to determine a specimen wherein enzymatic activities are extremely weak. In the latter method, the acceptor or the reaction product is labeled with ¹²⁵I and, therefore, the acceptor or the reaction product is limited to a glycoprotein. It is considered that, since structures of sugar chains of almost all of glycoproteins have not yet been entirely clarified, in general, specific determination of activities of a particular glycosyltransferase is difficult in so far as a glycoprotein is used. In addition, there is a defect in that the reaction product and an endogenous product of the same structure as the reaction product in the specimen cannot be differentiated according to this method, the endogenous product in the specimen affects the results of determination.

The present inventor has intensively studied in order to eliminate the problems in the above conventional methods and to find a simple, more precise and more specific method for determination of glycosyltransferase activities which is suitable for medical and clinical tests such as blood grouping, diagnosis of cancer and the like. As a result, it has been found that the above problems can be solved by a novel method for determination of activities of glycosyltransferases which comprises:
(a) using a sugar acceptor wherein an oligosaccharide is linked to an insoluble carrier or a protein; and
(b) using an antibody or lectin specifically binding to a reaction product of a sugar donor and a sugar acceptor with a glycosyltransferase, or using this antibody and the antibody specifically binding to the protein bound to a oligosaccharide (as shown (a)) in order to conduct the determination according to sandwich technique.

Japanese Patent Kokai No. 1-260364 published on 17.10.89 (i.e. after the priority date of the present application) discloses a determination method of glycosyltransferase activities which comprises reacting a specimen containing a glycosyltransferase to be determined and a sugar which is a substrate of the glycosyltransferase with an immobilized acceptor complex sugar wherein the acceptor complex sugar is linked to an insoluble carrier. The glycosyltransferase in the specimen produces a sugar chain specific for the glycosyltransferase on the insoluble carrier. This reaction product is bound to a labeled antibody which is obtained by labeling a monoclonal antibody to the structure of the sugar chain produced with a labeling agent, and then determining activities of the labeling agent bound to the insoluble carrier. However, the sugar acceptor used and determination method of enzymatic activity by measuring the amount of the reaction product in this method are different from those of the above novel method. Particularly, the sandwich technique as described in the above (b) is not suggested by this determination method.

### OBJECTS OF THE INVENTION

One object of the present invention is to provide a method for the determination of glycosyltransferase activities while avoiding the above disadvantages in the conventional methods.

Another object of the present invention is to provide a blood grouping method using the method for the determination of glycosyltransferase activities of the present invention.

Still another object of the present invention is to provide a method for the diagnosis of cancer using the method for the determination of glycosyltransferase activities of the present invention.

These objects as well as other objects and advantages of the present invention will become apparent to those skilled in the art from the following description with reference to the attached drawings.

### BRIEF DESCRIPTION OF THE DRAWING

Fig. 1 is a calibration curve illustrating the relation between the amount (pmol) of A antigen and the radioactivity (cpm x 10⁻³) used in Example 1 hereinafter.

Fig. 2 is a calibration curve illustrating the relation between the amount (pmol) of B antigen and the radioactivity (cpm x 10⁻³) used in Example 2 hereinafter.

Fig. 3 is a calibration curve illustrating the relation between the amount (pmol) of A antigen and the absorbance at 420 nm used in Example 3 hereinafter.

Fig. 4 is a calibration curve illustrating the relation between the amount (pmol) of B antigen and the absorbance at 420 nm used in Example 4 hereinafter.

Fig. 5 shows α1→3 fucosyltransferase activities in sera tested in Example 5 hereinafter.

Fig. 6 shows α1→3 fucosyltransferase activities in sera tested in Example 6 hereinafter.

### SUMMARY OF THE INVENTION

The first aspect of the present invention is to provide a method for the determination of activities of glycosyltransferases which comprises reacting a sugar donor of a non-labeled sugar nucleotide and a sugar acceptor, which is an oligosaccharide linked to an insoluble carrier, with a specimen to obtain an immobilized reaction product, rinsing out an endogenous product of the same structure as the reaction product in the specimen, and determining glycosyltransferase by measuring of the amount of the immobilized reaction product.

The second aspect of the present invention is to provide a method for the determination of activities of glycosyltransferases according to sandwich technique which comprises reacting a specimen with a reaction system composed of a sugar donor of a non-labeled sugar nucleotide and a sugar acceptor, which is an oligosaccharide linked to a protein, to obtain a reaction product, immobilizing the reaction product with an insoluble antibody which recognizes the protein portion of the reaction product, rinsing out an endogenous product of the same structure as the reaction product in the specimen, adding an antibody or lectin which recognizes a sugar portion produced by the reaction and determining glycosyltransferase by measuring the amount of the reaction product, said antibody or lectin binding to neither the sugar donor nor the sugar acceptor to which the sugar from the donor is not yet transferred.

The present invention also provides a method for blood grouping which comprises determining glycosyltransferase activities in a specimen by either the above method of the first aspect or that of the second aspect.

Further, the present invention provides a method for the diagnosis of cancer which comprises determining glycosyltransferase activities in a specimen by either the above method of the first aspect or that of the second aspect.

### DETAILED DESCRIPTION OF THE INVENTION

As described above, various methods have been proposed to determine the activities of glycosyltransferases by reacting a sugar donor of a sugar nucleotide with a sugar acceptor in the presence of a glycosyltransferase, separating the reaction product and determining the amount thereof.

However, in the method of the present invention, since a reaction product can be separated from a reaction system in an immobilized state, the reaction product can be readily handled and, in addition, an interference from endogenous product of the same structure as the reaction product in a specimen can be reduced. Further, the amount of the reaction product can be determined by various methods.

More particularly, according to the first aspect of the present invention, activities of glycosyltransferases are determined by using a non-labeled sugar nucleotide as a sugar donor, reacting the sugar donor and a sugar acceptor, wherein an oligosaccharide is linked to an insoluble carrier, with the specimen to obtain a immobilized reaction product. An endogenous product of the same structure as the reaction product in the specimen is rinsed out. A ¹²⁵I-labeled antibody or lectin, or an enzyme-labeled antibody or lectin is added to specifically react with the immobilized reaction product. Then, according to a conventional technique, the radioactivity of the immobilized reaction product or the degree of color development due to a pigment formed by the enzyme used as the labeling agent is determined.

Activities of glycosyltransferases can also be determined by using a non-labeled sugar nucleotide as a sugar donor, reacting the sugar donor and an immobilized acceptor, wherein the above sugar acceptor is linked to an insoluble carrier, with the specimen to obtain an immobilized reaction product. A non-labeled antibody is added to specifically react with the immobilized reaction product, and then the amount of the antibody bound is determined by a conventional enzyme-labeled antibody technique utilizing a secondary antibody to the antibody to determine the glycosyltransferase activity by measuring of the amount of the immobilized reaction product.

Or, activities of glycosyltransferases are determined by using a non-labeled sugar nucleotide as a sugar donor, reacting the sugar donor and a sugar acceptor, wherein an oligosaccharide is linked to an insoluble carrier, with a specimen to obtain an immobilized reaction product, adding a biotinated antibody specifically reacting with the resulting immobilized reaction product and determining activity of the immobilized reaction product by an avidinated enzyme according to a conventional technique.

In the second aspect of the present invention, determination of activities of glycosyltransferases is carried out by, for example, using a non-labeled sugar nucleotide as a sugar donor, reacting the donor and a sugar acceptor, wherein an oligosaccharide is linked to a protein, with the specimen. The resulting reaction product is immobilized with an insolubilized antibody specifically binding to the protein side thereof, and an endogenous product of the same structure as the reaction product in the specimen is rinsed out. Glycosyltransferase activity in the specimen is determined by measuring of the immobilized reaction product according to a conventional sandwich technique, forming a sandwich with an antibody specifically binding to a sugar portion produced. Of course, in the second aspect of the present invention, it is also possible to use a ¹²⁵I-labeled or enzyme-labeled antibody as well as to use biotin and avidin as a secondary antibody as in the first aspect of the present invention.

The sugar nucleotides used in the present invention includes GDP-fucose, UDP-N-acetylgalactosamine, UDP-N-acetylglucosamine, UDP-galactose, CMP-N-acetyl-neuraminic acid, GDP-mannose and the like.

Oligosaccharides, glycolipids, glycoproteins and the like are used as the sugar acceptor. Examples of the insoluble carrier which immobilizes these sugar acceptors include inorganic carriers such as silica, zirconia, metals and the like; and organic carriers such as synthetic polymers, natural polysaccharides and the like. They can be used alone or in combination thereof.

These carriers are in the shape of particles, spheres, walls of containers and the like. The insoluble sugar acceptor can be prepared by binding the carrier to the sugar acceptor chemically or physically according to the conventional method.

Examples of sugar acceptors include SYNSORB® (manufactured by CHEMBIOMED) which are insoluble carriers linked with sugar chains such as N-acetyllactosamine (Gal β1→4GlcNAc), lactose (Gal β1→4Glc), Lewis c (Gal β1→3GlcNAc), H antigen type I chain (Fuc α1→2 Gal β1→3GlcNAc), H antigen type II chain (Fuc α1→2 Gal β1→4GlcNAc), N-acetylgalactosamine (GalNAc), T type antigen (Gal β1→3GalNAc), Lewis a (Gal β1→3[Fuc α1→4]GlcNAc), Lewis X (Gal β1→4[Fuc α1→3]GlcNAc) and the like.

As for the protein to which an oligosaccharide is linked, a protein which is not present in the human blood (specimen) is preferred. For example, there can be used a protein obtained from other animals such as BSA (bovine serum albumin).

The glycosyltransferases to be determined by the method of present invention include fucosyltransferase, galactosyltransferase, N-acetylglucosaminyltransferase, sialyltransferase, mannosyltransferase, N-acetylgalactosaminyltransferase and the like.

Examples of the reaction product formed by reaction of these enzymes with the above sugar donors and sugar acceptors include insoluble carriers linked with sugar chains such as A type antigen (GalNAc α1→3 [Fuc α1→2] Gal), B type antigen (Gal α1→3 [Fuc α1→2] Gal), Lewis a type antigen (Gal β1→3 [Fuc α1→4] GlcNAc), Lewis b type antigen (Fuc α1→2Gal β1→3 [Fuc α1→4] GlcNAc), Lewis X (Gal β1→4[Fuc α1→3] GlcNAc), Lewis Y (Fuc α1→2 Gal β1→4[Fuc α1→3]GlcNAc), sialyl-Lewis X (NeuAc α2→3Gal β1→4[Fuc α1→3]GlcNAc), sialyl-Lewis a (NeuAc α2→3Gal β1→3[Fuc α1→4]GlcNAc), sialyl Tn antigen (NeuAc α2→6GalNAcα), T antigen (Gal β1→3GalNAc), sialyl T antigen (NeuAc α2→3 Gal β1→3GalNAc), Gal β1→3 [GlcNAc β1→6]GalNAc, GlcNAc β1→6GalNAc and the like.

Examples of substances specifically reacting with only the reaction product of the present invention include an antibody and lectin. The requisite for these specifically reacting substances is that they are neither binding to a sugar nucleotide which is the donor (sugar donor) nor the sugar acceptor to which the sugar from the donor is not yet transferred, but they must be specifically bound to only the sugar acceptor to which the sugar has already been transferred.

The antibody can be obtained by immunizing with a glycoprotein, glycolipid, oligosaccharide or the like which has a sugar chain of the same structure as the reaction product produced by a particular glycosyltransferase at its terminal end. In general, a monoclonal antibody is used as the specifically binding antibody because it is more advantageous in comparison with a polyclonal antibody in view of binding specificity. For example, there are various antibodies such as anti-A antibody to A type antigen, anti-B antibody to B type antigen and the like. As described above, each antibody can be determined by the radioactivity of ¹²⁵I according to the conventional technique, or by the degree of color development of a pigment produced by the enzyme used as a labeling agent according to a conventional enzyme-labeled antibody technique. In addition, a non-labeled antibody and a labeled secondary antibody can be used. Further, in the case of using a glycoprotein as the sugar acceptor, determination can be carried out by a conventional sandwich technique, wherein the antibody recognizing the sugar produced and the antibody recognizing the protein side are used.

The method of the present invention can be used for the determination of various glycosyltransferase activities in various specimens such as body fluid, for example, blood, serum, plasma and the like.

Particularly, the method of the present invention can be used in blood grouping and the diagnosis of cancer. More particularly, the determination of blood group specific glycosyltransferases can be employed for blood grouping. That is, determinations can be made of activities of glycosyltransferases associated with blood group specific glycosyltransferases such as H substance synthesizing α(1→2) fucosyltransferase, A type substance synthesizing α(1→3) N-acetylgalactosaminyl-transferase, B type substance synthesizing α(1→3) galactosyltransferase, Le type substance synthesizing α(1→4) fucosyltransferase, T type substance synthesizing β(1→3) galactosyltransferase and the like. In the diagnosis of cancer, activities of α(1→3) fucosyltransferase, α(2→3) sialyltransferase, α(2→6) sialyltransferase, α(2→8) sialyltransferase, β(1→3) N-acetylglucosaminyltransferase, β(1→4) galactosyltransferase, β1→6N-acetylglucosaminyltransferase, β1→3 galactosyltransferase and the like can be determined.

According to the present invention, when a ¹²⁵I-labeled antibody is used for determination of activities of the glycosyltransferase, a gamma-ray counter, which is a general-purpose counter and simple, can be used instead of a liquid scintillation counter. Furthermore, by using a non-radioisotope-labeled antibody, determination can be readily carried out without using special facilities.

Regarding specificity of the reaction system, since an antibody or lectin specifically reacting with the reaction product is used, the reaction system is superior in specificity. At the same time, since determination is carried out by using the acceptor in the immobilized state or immobilizing only the enzymatic reaction product, interference from endogenous products of the same structure as the reaction product in the specimen can be reduced. In this respect, the method of the present invention is also superior in specificity. Thus, according to the present invention, a simple, more precise quantitative method for determination of activities of blood group specific glycosyltransferases and cancer related-glycosyltransferases, for example, can be established with improved specificity and, by utilizing this method, variant blood grouping which has hitherto been difficult to conduct can be readily carried out.

The following Examples further illustrate the present invention in detail but are not to be construed to limit the scope thereof.

### Example 1

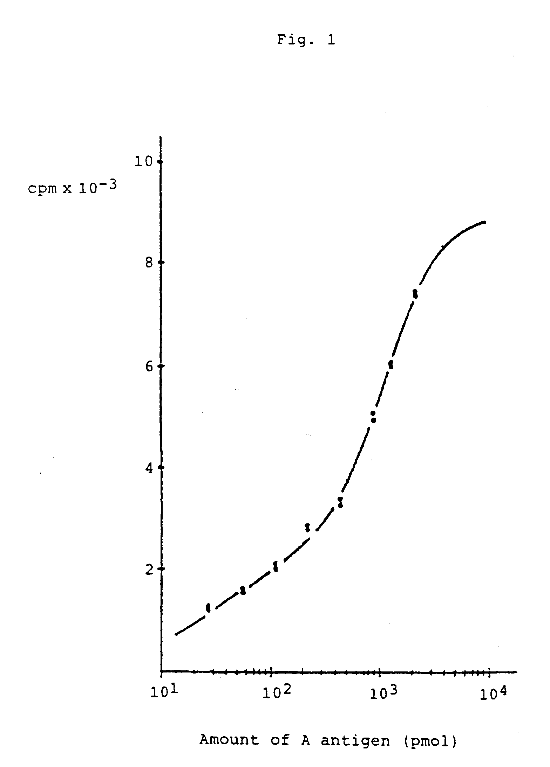

Beads of SYNSORB H® (manufactured by CHEMBIOMED) bonded with H (type II chain) hapten (Fuc α1→2Gal β1→4GlcNAc β→R) (17.4 nmol hapten/20 mg beads) used as a sugar acceptor, UDP-N-acetylgalactosamine (30 nmol), 100 µl of A buffer solution (GALSERVE AB®, manufactured by Sumitomo Seika Chemicals Co., Ltd., Japan) and 100 µl of serum (plasma) to be tested were added together and the mixture was incubated in a test tube at 37°C for 4 hours and the beads were then washed three times with 2 ml of physiological saline solution. To this was added 100 µl of ¹²⁵I-labeled purified anti-A monoclonal antibody (specific activity: 0.015 µCi/µg) and 100 µl of buffer (0.15 M PBS, pH 7.2, 0.02% NaN₃, 0.25% BSA). After incubating the mixture at room temperature for 30 minutes, the beads were washed again three times with 2 ml of physiological saline solution and the radioactivity of the bounded antibodies was determined by a gamma-ray counter. A calibration curve of A antigen as shown in Fig. 1 was prepared by using the beads of SYNSORB A® (amount of A antigen: 0.38 nmol/mg) as a standard substance and, according to the same manner as described above, adding ¹²⁵I-labeled purified anti-A monoclonal antibody and determining the amount of the antigen based on the radioactivity of the bound antibody. A specific glycosyltransferase activities in respective sera (plasmas) (amount of A antigen synthesized per 1 ml of the sample per one hour, pmol) determined are as follows.

| Samples | Enzymatic activities (pmol) |
|---|---|
| A₁ (n = 20) | 3059 ± 929 |
| A₁ B (n = 20) | 2657 ± 487 |
| Aᵢₙₜ (n = 3) | 1360 ±1154 |
| A₂ (A₂ B) (n = 6) | 623 ± 379 |
| A₃ (A₂ B) (n = 6) | 70 ± 53 |
| B (n = 20) | 0 |
| O (n = 20) | 0 |

The above results show that the activities of A specific glycosyltransferase which are present only in A type serum (plasma) can be determined in a short period of time and, at the same time, regarding A variant type, determination of the enzymatic activity which has hitherto been difficult to conduct can be also readily carried out in the same manner.

### Example 2

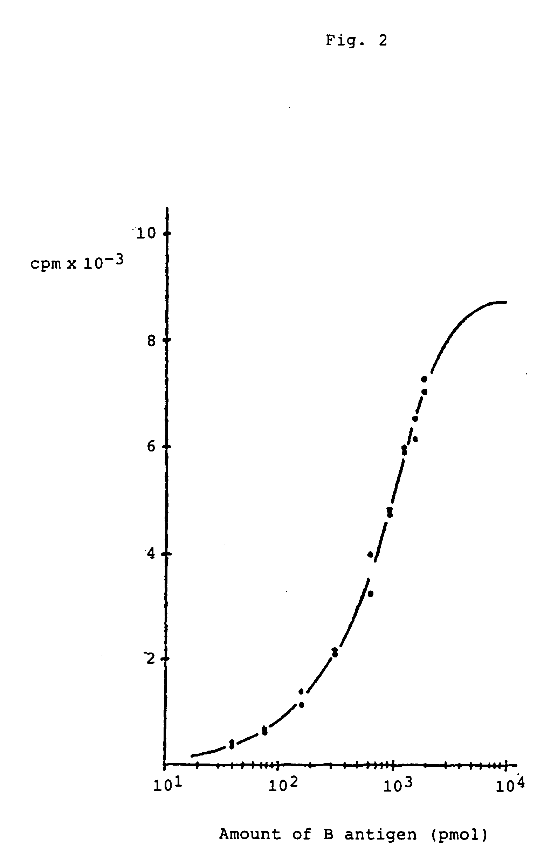

Beads of SYNSORB H® (manufactured by CHEMBIOMED) bonded with H (type II chain) hapten (Fuc α1→2Gal β1→4GlcNAc β→R) (17.4 nmol hapten/20 mg beads) used as a sugar acceptor, UDP-galactose (30 nmol), 100 µl of B buffer solution (GALSERVE AB®, manufactured by Sumitomo Seika Chemicals Co., Ltd., Japan) and 100 µl of serum (plasma) to be tested were added together and the mixture was incubated in a test tube at 37°C for 4 hours and the beads were washed three times with 2 ml of physiological saline solution. To this was added 100 µl of ¹²⁵I-labeled purified anti-B monoclonal antibody (specific activity: 0.015 µCi/µg) and 100 µl of buffer (0.15 M PBS, pH 7.2, 0.02% NaN₃, 0.25% BSA). After incubating the mixture at room temperature for 30 minutes, the beads were washed again three times with 2 ml of physiological saline solution and the radioactivity of the bound antibody was determined by a gamma-ray counter. A calibration curve as shown in Fig. 2 of B antigen was prepared by using SYNSORB B® (amount of B antigen: 0.31 nmol/mg) as a standard substance and, in the same manner as described above, adding ¹²⁵I-labeled purified anti-B monoclonal antibody and determining the amount of the antigen based on the radioactivity of the bound antibody. B specific glycosyltransferase activities in respective sera (plasmas) (amount of B antigen synthesized per 1 ml of the sample per one hour, pmol) are as follows.

| Samples | Enzymatic activities |
|---|---|
| B (n = 20) | 713 ± 209 |
| AB (n = 20) | 591 ± 172 |
| B₂ (AB₂) (n = 4) | 352 ± 275 |
| B₃ (AB₃) (n = 7) | 49 ± 40 |
| Bₘ (ABₘ) (n = 7) | 150 ± 73 |
| A (n = 20) | 0 |
| O (n = 20) | 0 |

The above results show that the activities of B specific glycosyltransferase which are present only in B type serum (plasma) can be determined in a short period of time and, at the same time, regarding B subtype and variant type, determination of enzymatic activities which has hitherto been difficult to conduct can be also readily carried out according to the same manner.

### Example 3

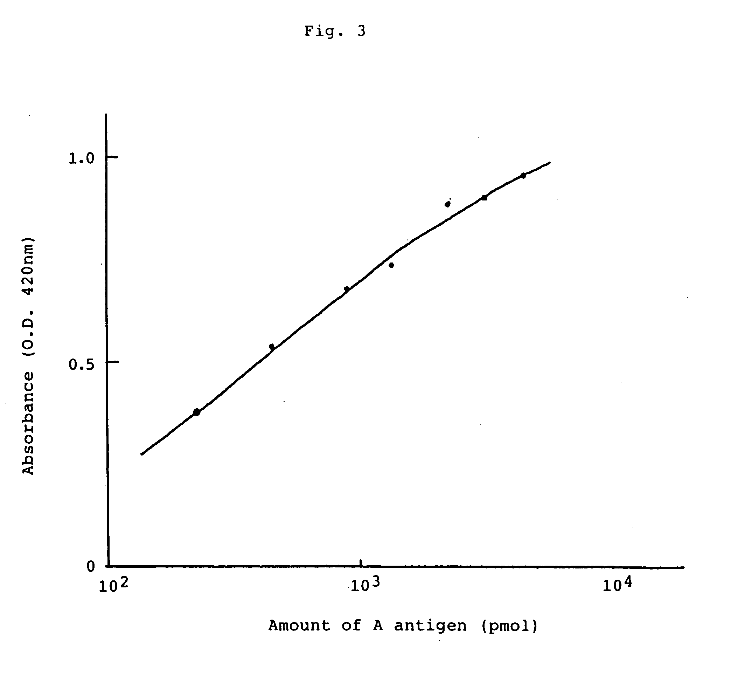

Beads of SYNSORB H® (manufactured by CHEMBIOMED) bonded with H (type II chain) hapten (Fuc α1→2Gal β1→4GlcNAc β→R) (17.4 nmol hapten/20 mg beads) used as an acceptor, UDP-N-acetylgalactosamine (30 nmol), A buffer solution (100 µl, GALSERVE AB® manufactured by Sumitomo Seika Chemical Co., Ltd., Japan) and plasma to be tested (100 µl) were added together and the mixture was incubated at 37°C for 12 to 18 hours. Then, the beads were washed three times with distilled water (2 ml). To the beads was added a solution (200 µl) of anti-A monoclonal antibody purified with affinity chromatography (SYNNAF A® manufactured by CHEMBIOMED) diluted with PBS (pH 7.0) containing 0.5% of gelatin and the mixture was incubated at room temperature for 1 hour. The beads were again washed three times with distilled water (2 ml). To the beads was added a solution (200 µl) of peroxidase labeled anti-mouse IgM antibody purified by affinity chromatography (manufactured by TAGO) diluted with PBS containing 0.5% of gelatin and the mixture was incubated at room temperature for 2 hours. The beads were washed three times with distilled water (2 ml). To the beads was added ABTS-H₂O₂ solution (200 µl) to develop color, oxalic acid was added thereto and the mixture was subjected to colorimetry at optical density of 420 nm. In order to determine the amount of the antigen formed quantitatively, a calibration curve of A antigen as shown in Fig. 3 was prepared by using SYNSORB A® (0 to 4.5 nmol/0 to 20 mg beads) linked with A hapten (GalNAc α1→3[Fuc α1→2]-Gal β→R) as a standard substance and, in the same manner as described above, adding the primary and secondary antibodies, incubating the mixture and subjecting to colorimetry to determine the amount of A antigen.

When A specific glycosyltransferase activities in plasmas of various types including subtypes were examined, enzymatic activities were specifically detected in A and AB types and no A specific glycosyltransferase activity was detected in O and B types. Enzymatic activities of respective plasmas (the amount of A antibody produced per 1 ml of plasma per 1 hour) are as follows:

| Plasmas | Enzymatic activities (pmol) |
|---|---|
| A₁ (n = 20) | 2278 ± 669 |
| A₁B (n = 20) | 2315 ± 682 |
| A₂ (A₂B) (n = 8) | 1559 ± 624 |
| A₃ (n = 4) | 444 ± 394 |

The above results show that, as determination by RIA using ¹²⁵I-labeled anti-A in Example 1, an amount of activity of A specific glycosyltransferase which is present only in A type serum (plasma) can be determined specifically and, at the same time, regarding A subtype and variant type, determination of enzymatic activities, which has hitherto been difficult to conduct, can be simply carried out according to the same manner.

### Example 4

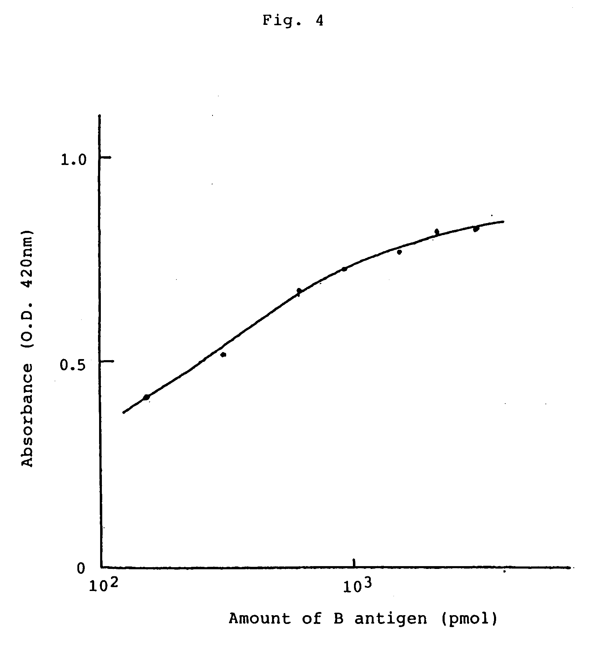

Beads of SYNSORB H® (manufactured by CHEMBIOMED) bonded with H (type II chain) hapten (17.4 nmol hapten/20 mg beads) used as an acceptor, UDP-galactose (30 nmol), B buffer solution (100 µl, GALSERVE AB® manufactured by Sumitomo Seika Chemicals Co., Ltd., Japan) and plasma to be tested (100 µl) were added together and the mixture was incubated at 37°C for 12 to 18 hours. After incubation, the beads were washed three times with distilled water (2 ml). To the beads was added a solution (200 µl) of anti B monoclonal antibody purified by affinity chromatography (SYNNAF B® manufactured by CHEMBIOMED) diluted with PBS containing 0.5% of gelatin, the mixture was incubated at room temperature for 1 hour and then the beads were washed three times with distilled water (2 ml). Furthermore, to the beads was added a solution (200 µl) of peroxidase labeled anti-mouse IgM antibody purified by affinity chromatography (TAGO) diluted with PBS containing 0.5% of gelatin and the mixture was incubated at room temperature for 2 hours. The beads were washed three times with distilled water (2 ml). ABTS-H₂O₂ solution (200 µl) was added to the beads to develop color, oxalic acid was added thereto and the mixture was subjected to colorimetry at the optical density of 420 nm. In order to determine the antigen formed quantitatively, a calibration curve of the amount of B antigen as shown in Fig. 4 was prepared by using SYNSORB B® linked with B hapten (Gal α1→3[Fuc α1→2]Gal β→R) (0 to 3.1 nmol/0 to 20 mg beads) as a standard substance and, according to the same manner as described above, adding the primary and secondary antibodies, incubating the mixture and subjecting to colorimetry.

When B specific glycosyltransferase activities in plasmas of various types including subtypes were examined, B type enzymatic activities were specifically detected in B and AB types and no B specific glycosyltransferase activity was detected in O and A types. Enzymatic activities of respective plasmas (the amount of A antibody produced per 1 ml of plasma per 1 hour) are as follows:

| Plasmas | Enzymatic activities (pmol) |
|---|---|
| B (n = 20) | 1465 ± 578 |
| AB (n = 15) | 1516 ± 426 |
| B_{weak} (n = 5) | 732 ± 607 |
| B₃ (n = 3) | 242 ± 197 |
| Bₘ (n = 6) | 689 ± 374 |

The above results show that, as determination by RIA using ¹²⁵I-labeled anti-B in Example 2, the amount of activity of B specific glycosyltransferase which is present only in B type serum (plasma) can be determined specifically and, at the same time, regarding B subtype and variant type, determination of enzymatic activities, which has hitherto been difficult to conduct, can be simply carried out according to the same manner.

### Example 5

Beads of SYNSORB H® type II chain (8.7 nmol hapten/10 mg beads, manufactured by CHEMBIOMED) used as a sugar acceptor, GDP-fucose (14 nmol, manufactured by Sumitomo Seika Chemicals Co., Ltd.), ATP (0.4 µmol, Sigma Chemical Company), MnCl₂ (2 µmol, WAKO PURE CHEMICAL INDUSTRIES LTD.), HEPES buffer (100 µl, pH 7.0) and serum to be tested (100 µl) were added together and the mixture was incubated in a test tube at 37°C for 40 hours. The beads were washed five times with physiological saline solution (2 ml). ¹²⁵I-labeled anti-Lewis Y antibody (specific activity: 0.9 µCi/µg, 200 µl, about 50,000 cpm) was added to the reaction tube and the mixture was incubated at room temperature for 2 hours. The beads were again washed five times with physiological saline (2 ml) and radioactivity of the bounded antibody was determined with a gamma-ray counter.

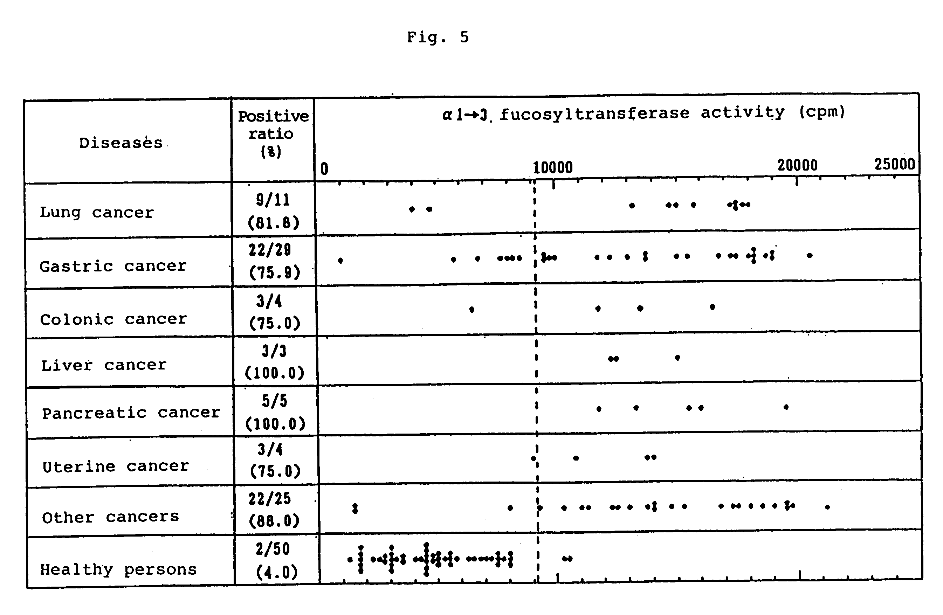

The results obtained by the determination of Lewis Y synthetase activities (α1→3 fucosyltransferase activities) of sera of healthy normal persons and patients with various cancers are shown in Fig. 5. These results show that the determination of Lewis Y synthetase can be conducted by the method of the present invention as well as the usefulness of the present invention in the diagnosis of cancer.

### Example 6

Glycoprotein of SYNTAGEN H® type II chain (6.3 nmol hapten/0.5 µg, manufactured by CHEMBIOMED), an oligosaccharide linked with bovine serum albumin (BSA), used as a sugar acceptor, GDP-fucose (15 nmol, Sumitomo Seika Chemicals Co., Ltd.), ATP (0.4 µmol, Sigma Chemical Company), MnCl₂ (2 µmol, WAKO PURE CHEMICAL INDUSTRIES LTD.), HEPES buffer (200 µl, pH 7.0) and serum to be tested (100 µl) were added together and the mixture was incubated in a test tube at 37°C for 40 hours. One anti-BSA antibody-immobilized polystylene bead was added and the mixture was further incubated at 37°C for 2 hours. The bead was washed three times with distilled water (2 ml). ¹²⁵I-labeled anti-Lewis Y antibody (specific activity: 0.9 µCi/µg, 200 µl, about 100,000 cpm) was added and the mixture was incubated at room temperature for 2 hours. The bead was again washed three times with distilled water (2 ml) and the radioactivity of the bounded antibody was determined by a gamma-ray counter.

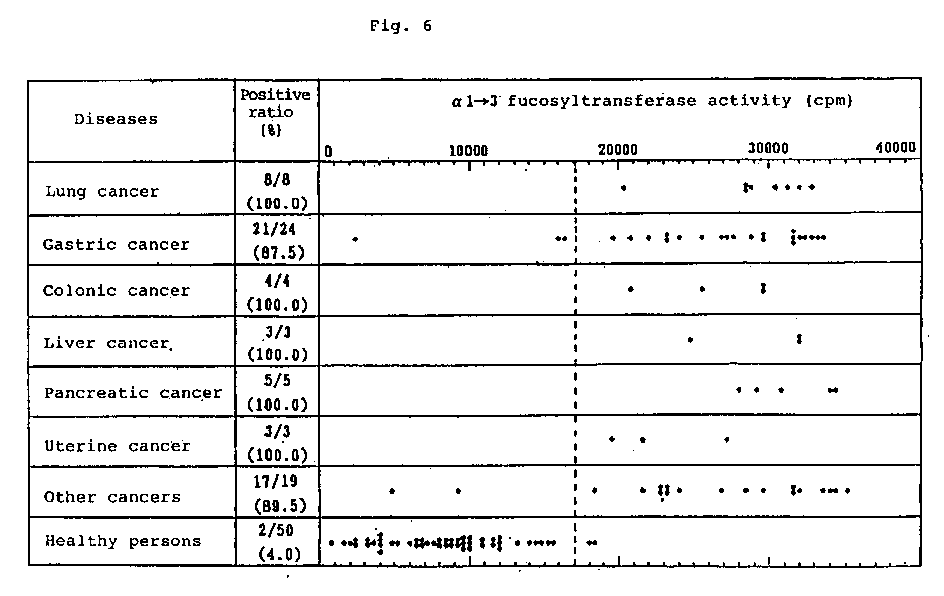

The results obtained by determination of Lewis Y synthetase activities (α1→3 fucosyltransferase activities) in sera of healthy normal persons and patients with various cancers are shown in Fig. 6. The results show that, as the above method of the present invention using an insolubilized oligosaccharide, is also useful for the diagnosis of cancer.

### Example 7

Glycoprotein of SYNTAGEN® Lewis C chain (5.8 nmol/hapten 0.5 µg, manufactured by CHEMBIOMED), an oligosaccharide linked with BSA, used as a sugar acceptor, CMP-N-acetylneuraminic acid (30 nmol, Sigma Chemical Company), ATP (0.4 µmol, Sigma Chemical Company), MnCl₂ (2 µmol, WAKO PURE CHEMICAL INDUSTRIES LTD.), HEPES buffer (200 µl, pH 7.0) and serum to be tested were added together and the mixture was incubated in a test tube at 37°C for 40 hours. One anti-BSA antigen-immobilized polystylene bead was added and the mixture was further incubated at 37°C for 2 hours and the bead was washed three times with distilled water (2 ml). To this was added ¹²⁵I-labeled anti-sialyl α2→3 Lewis C antibody (specific activity: 5.2 µCi/µg, 200 µl, about 100,000 cpm) and the mixture was incubated at room temperature for 2 hours. The bead was washed three times with distilled water and the radioactivity of the bounded antigen was determined with a gamma-ray counter.

In the results, α2→3 sialyltransferase (I) activities of sera of patients with cancer were significantly higher than those of healthy normal persons. This shows that the determination of α2→3 sialyltransferase activities by the method of the present invention is useful in the diagnosis of cancer.

### Example 8

Glycoprotein of Tn antigen (α-N-acetylgalactosaminylthreonine, GalNAc α1→O-Thr, 7.2 nmol hapten/0.5 µg), an oligosaccharide linked with BSA, used as a sugar acceptor, CMP-N-acetylneuraminic acid (30 nmol, Sigma Chemical Company), ATP (0.4 µmol, Sigma Chemical Company), MnCl₂ (2 µmol, WAKO PURE CHEMICAL INDUSTRIES LTD.), HEPES buffer (200 µl, pH 7.0) and serum to be tested (100 µl) were added together and the mixture was incubated in a test tube at 37°C for 40 hours. Then, one anti-BSA antibody-immobilized polyethylene bead was added and the mixture was further incubated at 37°C for 2 hours. The bead was washed three times with distilled water (2 ml) and ¹²⁵I labeled anti sialyl α2→6 Tn antibody (specific activity: 5.1 µCi/µg, 200 µl, about 100,000 cpm) was added thereto. The mixture was incubated at room temperature for 2 hours, the bead was washed three times with distilled water (2 ml) and the radioactivity of the bounded antibody was determined with a gamma-ray counter.

In the results, α2→6 sialyltransferase (III) activities of sera of patients with cancer were significantly higher than those of healthy normal persons. This shows that determination of α2→6 sialyltransferase activities by the method of the present invention is useful for the diagnosis of cancer.

## Claims

1. A method for the determination of activities of glycosyltransferases which comprises reacting a sugar donor of a non-labeled sugar nucleotide and a sugar acceptor, which is an oligosaccharide, a glycolipid or a glycoprotein linked to an insoluble carrier, with a specimen to obtain an immobilized reaction product, rinsing out an endogenous product of the same structure as the reaction product in the specimen and determining glycosyltransferase by measuring the amount of the immobilized reaction product with an antibody or lectin which specifically reacts with the reaction product, said antibody or lectin neither binding to the sugar donor nor to the sugar acceptor to which the sugar from the donor is not yet transferred.

2. A method according to claim 1, wherein ¹²⁵I-labeled antibody or lectin is used as the antibody or lectin specifically reacting with the immobilized reaction product and the radioactivity of the immobilized reaction product is determined.

3. A method according to claim 1, wherein an enzyme-labeled antibody or lectin is used as the antibody or lectin specifically reacting with the immobilized reaction product and the degree of color development due to a pigment formed by the labeling enzyme is determined.

4. A method according to claim 1, wherein a non-labeled antibody is used as the antibody specifically reacting with the immobilized reaction product and the amount of the bound antibody is determined by a secondary antibody to the antibody.

5. A method according to claim 1, wherein a biotinated antibody or lectin is added as the antibody or lectin specifically reacting with the immobilized reaction product and the amount of the immobilized reaction product is determined by using an avidinated enzyme.

6. A method for blood grouping which comprises determining glycosyltransferase activities in a specimen by reacting a sugar donor of a non-labeled sugar nucleotide and a sugar acceptor, which is an oligosaccharide linked to an insoluble carrier, with the specimen to obtain an immobilized reaction product, rinsing out an endogenous product of the same structure as the reaction product in the specimen, and determining glycosyltransferase by measuring the amount of the immobilized reaction product with an antibody or lectin which specifically reacts with the reaction product, said antibody or lectin binding to neither the sugar donor nor the sugar acceptor to which the sugar from the donor has not yet been transferred.

7. A method for the diagnosis of cancer which comprises determining glycosyltransferase activities in a specimen by reacting a sugar donor of a non-labeled sugar nucleotide and a sugar acceptor, which is an oligosaccharide linked to an insoluble carrier, with the specimen to obtain an immobilized reaction product, rinsing out an endogenous product of the same structure as the reaction product in the Specimen, and determining glycosyltransferase by measuring the amount of the immobilized reaction product with an antibody or lectin which specifically reacts with the reaction product, said antibody or lectin binding to neither the sugar donor nor the sugar acceptor to which the sugar from the donor has not yet been transferred.

8. A method for the determination of activities of glycosyltransferases according to sandwich technique which comprises reacting a specimen with a reaction system composed of a sugar donor of a non-labeled sugar nucleotide and a sugar acceptor, which is an oligosaccharide linked to a protein not present in human body fluid, to obtain a reaction product, immobilizing the reaction product with an insoluble antibody which recognizes the protein portion of the reaction product, rinsing out an endogenous product of the same structure as the reaction product in the specimen, adding an antibody or lectin which recognizes a sugar portion produced by the reaction and determining glycosyltransferase by measuring the amount of the reaction product, said antibody or lectin not being bound to the sugar donor and the sugar acceptor to which the sugar from the donor is not yet transferred.

9. A method according to claim 8, wherein the antibody or lectin which recognizes a sugar portion produced is an ¹²⁵I-labeled antibody or lectin and the radioactivity thereof is determined.

10. A method according to claim 8, wherein the antibody or lectin which recognizes a sugar portion produced is an enzyme-labeled antibody or lectin and the degree of color development due to a pigment formed by the labeling enzyme is determined.

11. A method according to claim 8, wherein the antibody which recognizes a sugar portion produced is a non-labeled antibody and the amount of the bounded antibody is determined by using a secondary antibody to the antibody.

12. A method according to claim 8, wherein the antibody or lectin which recognizes a sugar portion produced is a biotinated antibody or lectin and the amount of the immobilized reaction product is determined by using an avidinated enzyme.

13. A method for blood grouping which comprises determining glycosyl-transferase activities in a specimen according to sandwich technique by reacting the specimen with a reaction system composed of a sugar donor of a non-labeled sugar nucleotide and a sugar acceptor, which is an oligosaccharide linked to a protein not present in human body fluid, to obtain a reaction product, immobilizing the reaction product with an insoluble antibody which recognizes the protein portion of the reaction product, rinsing out an endogenous product of the same structure as the reaction product in the specimen, adding an antibody or lectin which recognizes a sugar portion produced by the reaction and determining glycosyltransferase by measuring of the amount of the reaction product with an antibody or lectin which specifically reacts with the reaction product, said antibody or lectin binding to neither the sugar donor nor the sugar acceptor to which the sugar donor has not yet been transferred.

14. A method for the diagnosis of cancer which comprises determining glycosyltransferase activities in a specimen according to sandwich technique by reacting the specimen with a reaction system composed of a sugar donor of a non-labeled sugar nucleotide and a sugar acceptor, which is an oligosaccharide linked to a protein not present in human body fluid, to obtain a reaction product, immobilizing the reaction product with an insoluble antibody which recognizes the protein portion of the reaction product, rinsing out an endogenous product of the same structure as the reaction product in the specimen, adding an antibody or lectin which recognizes a sugar portion produced by the reaction and determining glycosyltransferase by measuring the amount of the reaction product with an antibody or lectin which specifically reacts with the reaction product, said antibody or lectin binding to neither the sugar donor nor the sugar acceptor to which the sugar donor has not yet been transferred.

## Patentansprüche

1. Verfahren zur Bestimmung von Aktivitäten von Glycosyltransferasen, umfassend das Reagieren eines Zuckerdonors, eines nichtmarkierten Zuckernukleotids und eines Zuckerakzeptors, der ein Oligosaccharid, ein Glycolipid oder ein Glycoprotein, gebunden an einen unlöslichen Träger ist, mit einer Probe, um ein immobilisiertes Reaktionsprodukt zu erhalten, das Auswaschen eines endogenen Produkts mit der gleichen Struktur wie das Reaktionsprodukt in der Probe und das Bestimmen der Glycosyltransferase durch Messen der Menge an immobilisiertem Reaktionsprodukt mit einem Antikörper oder Lectin, der oder das spezifisch mit dem Reaktionsprodukt reagiert, wobei der Antikörper oder das Lectin weder von dem Zuckerdonor noch dem Zuckerakzeptor, auf den noch kein Zucker von dem Donor übertragen wurde, gebunden werden.

2. Verfahren gemäß Anspruch 1, in dem ein ¹²⁵I-markierter Antikörper oder Lectin als spezifisch mit dem immobilisierten Reaktionsprodukt reagierender Antikörper oder reagierendes Lectin verwendet wird und die Radioaktivität des immobilisierten Reaktionsprodukts bestimmt wird.

3. Verfahren gemäß Anspruch 1, in dem ein enzymmarkierter Antikörper oder Lectin als spezifisch mit dem immobilisierten Reaktionsprodukt reagierender Antikörper oder reagierendes Lectin verwendet wird, und der Grad an Farbentwicklung aufgrund eines von dem markierenden Enzym gebildeten Pigments bestimmt wird.

4. Verfahren gemäß Anspruch 1, in dem ein nichtmarkierter Antikörper als spezifisch mit dem immobilisierten Reaktionsprodukt reagierender Antikörper verwendet wird, und die Menge an gebundenem Azitikörper durch einen sekundären Antikörper für den Antikörper bestimmt wird.

5. Verfahren gemäß Anspruch 1, in dem ein biotinierter Antikörper oder ein biotiniertes Lectin als spezifisch mit dem immobilisierten Reaktionsprodukt reagierender Antikörper oder reagierendes Lectin zugesetzt wird und die Menge an zugesetztem Reaktionsprodukt mit einem avidiniertem Enzym bestimmt wird.

6. Verfahren zur Bestimmung von Blutgruppen, umfassend das Bestimmen von Glycosyltransferase-Aktivitäten in einer Probe durch Reagieren eines Zuckerdonors, eines nichtmarkierten Zuckernukleotids und eines Zuckerakzeptors, der ein Oligosaccharid gebunden an einen unlöslichen Träger ist, mit einer Probe um ein immobilisiertes Reaktionsprodukt zu erhalten, das Auswaschen eines endogenen Produkts mit der gleichen Struktur wie das Reaktionsprodukt in der Probe und das Bestimmen der Glycosyltransferase durch Messen der Menge an immobilisiertem Reaktionsprodukt mit einem Antikörper oder Lectin, der oder das spezifisch mit dem Reaktionsprodukt reagiert, wobei der Antikörper oder das Lectin weder von dem Zuckerdonor noch von dem Zuckerakzeptor auf den der Zucker von dem Donor noch nicht übertragen wurde, gebunden wird.

7. Verfahren zur Diagnose von Krebs, umfassen das Bestimmen von Glycosyltransferase-Aktivitäten in einer Probe durch Reagieren eines Zuckerdonors, eines nichtmarkierten Zuckernukleotids und eines Zuckerakzeptors, der ein Oligosaccharid gebunden an einen unlöslichen Träger ist, mit einer Probe um ein immobilisiertes Reaktionsprodukt zu erhalten, das Auswaschen eines endogenen Produkts mit der gleichen Struktur wie das Reaktionsprodukt in der Probe und das Bestimmen der Glycosyltransferase durch Messen der Menge an immobilisiertem Reaktionsprodukt mit einem Antikörper oder Lectin, der oder das spezifisch mit dem Reaktionsprodukt reagiert, wobei der Antikörper oder das Lectin weder von dem Zuckerdonor noch von dem Zuckerakzeptor auf den der Zucker von dem Donor noch nicht übertragen wurde, gebunden wird.

8. Verfahren zur Bestimmung der Aktivitäten von Glycosyltransferasen nach der Sandwich-Technik umfassend das Reagieren einer Probe mit einem Reaktionssystem, zusammengesetzt aus einem Zuckerdonor eines nichtmarkierten Zuckernukleotids und eines Zuckerakzeptors, der ein Oligosaccharid gebunden an ein Protein ist, das nicht in der menschlichen Körperflüssigkeit vorhanden ist, um ein Reaktionsprodukt zu erhalten, das Immobilisieren des Reaktionsprodukts mit einem unlöslichen Antikörper, der den Proteinteil des Reaktionsprodukts erkennt, das Auswaschen eines endogenen Produkts mit der gleichen Struktur wie das Reaktionsprodukt aus der Probe, das Zugeben eines Antikörpers oder Lectins, der oder das den in der Reaktion gebildeten Zuckerteil erkennt und Bestimmen der Glycosyltransferase durch Messen der Menge an Reaktionsprodukt, wobei der Antikörper oder das Lectin nicht von dem Zuckerdonor und dem Zuckerakzeptor, auf den der Zucker von dem Donor noch nicht übertragen wurde, gebunden wird.

9. Verfahren gemäß Anspruch 8, in dem der Antikörper oder das Lectin, der oder das den gebildeten Zuckerteil erkennt, ein ¹²⁵I-markierter Antikörper oder Lectin ist, und dessen Radioaktivität bestimmt wird.

10. Verfahren gemäß Anspruch 8, in dem der Antikörper oder das Lectin, der oder das den gebildeten Zuckerteil erkennt, ein enzymmarkierter Antikörper oder Lectin ist, und der Grad an Farbentwicklung aufgrund eines von dem markierenden Enzym gebildeten Pigments bestimmt wird.

11. Verfahren gemäß Anspruch 8, in dem der Antikörper, der den gebildeten Zuckerteil erkennt, ein nichtmarkierter Antikörper ist, und die Menge an gebundenem Antikörper mit einem sekundären Antikörper für den Antikörper bestimmt wird.

12. Verfahren gemäß Anspruch 8, in dem der Antikörper oder das Lectin, der oder das den gebildeten Zuckerteil erkennt, ein biotinierter Antikörper oder Lectin ist und daß die Menge an immobilisierten Reaktionsprodukt mit einem avidinierten Enzym bestimmt wird.

13. Verfahren zur Bestimmung von Blutgruppen umfassend das Bestimmen von Glycosyltransferase-Aktivitäten in einer Probe nach der Sandwich-Technik durch Reagieren der Probe mit einem Reaktionssystem, zusammengesetzt aus einem Zuckerdonor eines nichtmarkierten Zuckernukleotids und einem Zuckerakzeptor, der ein Oligosaccharid, gebunden an ein Protein ist, das nicht in der menschlichen Körperflüssigkeit vorkommt, um ein Reaktionsprodukt zu erhalten, das Immobilisieren des Reaktionsprodukts mit einem unlöslichen Antikörper, der den Proteinteil des Reaktionsprodukts erkennt, das Auswaschen eines endogenen Produkts mit der gleichen Struktur wie das Reaktionsprodukt in der Probe, das Zugeben eines Antikörpers oder Lectins, der oder das den in der Reaktion gebildeten Zuckerteil erkennt und das Bestimmen der Glycosyltransferase durch Messen der Menge an Reaktionsprodukt mit einem Antikörper oder Lectin, der oder das spezifisch mit dem Reaktionsprodukt reagiert, wobei der Antikörper oder das Lectin weder von dem Zuckerdonor noch von dem Zuckerakzeptor auf den der Zucker vom Zuckerdonor noch nicht übertragen wurden gebunden wird.

14. Verfahren zur Diagnose von Krebs, umfassend das Bestimmen von Glycosyltransferase-Aktivitäten in einer Probe nach der Sandwich-Technik durch Reagieren der Probe mit einem Reaktionssystem zusammengesetzt aus einem Zuckerdonor eines nichtmarkierten Zuckernukleotids und eines Zuckerakzeptors, der ein Oligosaccharid, gebunden an ein Protein ist, das nicht in der menschlichen Körperflüssigkeit vorhanden ist, um ein Reaktionsprodukt zu erhalten, das Immobilisieren des Reaktionsprodukts mit einem unlöslichen Antikörper, der den Proteinteil des Reaktionsprodukts erkennt, das Auswaschen eines endogenen Produkts mit der gleichen Struktur wie das Reaktionsprodukt in der Probe, das Zugeben eines Antikörpers oder Lectins der oder das den in der Reaktion gebildeten Zuckerteil erkennt und das Bestimmen der Glycosyltransferase durch Messen der Menge an Reaktionsprodukt mit einem Antikörper oder Lectin der oder das spezifisch mit dem Reaktionsprodukt reagiert, wobei der Antikörper oder das Lectin weder von dem Zuckerdonor noch dem Zuckerakzeptor auf den der Zucker von dem Zuckerdonor noch nicht übertragen worden ist, gebunden wird.

## Revendications

1. Procédé de détermination d'activités de glycosyltransférase, qui comprend la réaction d'un donneur de sucre d'un nucléotide à sucre non marqué et d'un accepteur de sucre, qui est un oligosaccharide, un glycolipide ou une glycoprotéine lié à un support insoluble, avec un échantillon, pour obtenir un produit de réaction immobilisé, l'élimination de l'échantillon, par rinçage, d'un produit endogène de même structure que le produit de réaction, et la détermination de la présence de glycosyltransférase par mesure de la quantité de produit de réaction immobilisé avec un anticorps ou une lectine qui réagit de façon spécifique avec le produit de réaction, ledit anticorps ou ladite lectine ne se fixant ni sur le donneur de sucre, ni sur l'accepteur de sucre sur lequel le sucre du donneur n'a pas encore été transféré.

2. Procédé selon la revendication 1, dans lequel on utilise un anticorps ou une lectine marqué avec ¹²⁵I comme anticorps ou lectine réagissant de façon spécifique avec le produit de réaction immobilisé, et dans lequel on détermine la radioactivité du produit de réaction immobilisé.

3. Procédé selon la revendication 1, dans lequel on utilise un anticorps ou une lectine marqué par une enzyme comme anticorps ou lectine réagissant de façon spécifique avec le produit de réaction immobilisé et dans lequel on détermine le développement de couleur dû à un pigment formé par l'enzyme de marquage.

4. Procédé selon la revendication 1, dans lequel on utilise un anticorps non marqué comme anticorps réagissant de façon spécifique avec le produit de réaction immobilisé et dans lequel on détermine la quantité d'anticorps lié à l'aide d'un anticorps secondaire dirigé contre l'anticorps.

5. Procédé selon la revendication 1, dans lequel on ajoute un anticorps ou une lectine biotiné comme anticorps ou lectine réagissant de façon spécifique avec le produit de réaction immobilisé et dans lequel on détermine la quantité de produit de réaction immobilisé en utilisant une enzyme avidinée.

6. Procédé de détermination des groupes sanguins, qui comprend la détermination d'activités de glycosyltransférase dans un échantillon, par réaction d'un donneur de sucre d'un nucléotide à sucre non marqué et d'un accepteur de sucre, qui est un oligosaccharide lié à un support insoluble, avec l'échantillon, pour obtenir un produit de réaction immobilisé, l'élimination de l'échantillon, par rinçage, d'un produit endogène de même structure que le produit de réaction, et la détermination de la présence de glycosyltransférase par mesure de la quantité de produit de réaction immobilisé avec un anticorps ou une lectine qui réagit de façon spécifique avec le produit de réaction, ledit anticorps ou ladite lectine ne se fixant ni sur le donneur de sucre, ni sur l'accepteur de sucre sur lequel le sucre du donneur n'a pas encore été transféré.

7. Procédé de diagnostic du cancer, qui comprend la détermination d'activités de glycosyltransférase dans un échantillon, par réaction d'un donneur de sucre d'un nucléotide à sucre non marqué et d'un accepteur de sucre, qui est un oligosaccharide lié à un support insoluble, avec l'échantillon, pour obtenir un produit de réaction immobilisé, élimination de l'échantillon, par rinçage, d'un produit endogène de même structure que le produit de réaction et détermination de la présence de glycosyltransférase par mesure de la quantité de produit de réaction immobilisé avec un anticorps ou une lectine qui réagit de façon spécifique avec le produit de réaction, ledit anticorps ou ladite lectine ne se fixant ni sur le donneur de sucre, ni sur l'accepteur de sucre sur lequel le sucre du donneur n'a pas encore été transféré.

8. Procédé de détermination d'activités de glycosyltransférase selon la technique en sandwich, qui comprend la réaction d'un échantillon avec un système de réaction composé d'un donneur de sucre d'un nucléotide à sucre non marqué et d'un accepteur de sucre, qui est un oligosaccharide lié à une protéine non présente dans les fluides corporels humains, pour obtenir un produit de réaction, l'immobilisation du produit de réaction avec un anticorps insoluble qui reconnait la portion protéique du produit de réaction, l'élimination de l'échantillon, par rinçage, d'un produit endogène de même structure que le produit de réaction, l'addition d'un anticorps ou d'une lectine qui reconnait une portion sucre produite par la réaction, et la détermination de la présence de glycosyltransférase par mesure de la quantité de produit de réaction, ledit anticorps ou ladite lectine ne se fixant ni sur le donneur de sucre, ni sur l'accepteur de sucre sur lequel le sucre du donneur n'a pas encore été transféré.

9. Procédé selon la revendication 8, dans lequel l'anticorps ou la lectine qui reconnait une portion sucre produite est un anticorps ou une lectine marqué avec ¹²⁵I et dans lequel on détermine sa radioactivité.

10. Procédé selon la revendication 8, dans lequel l'anticorps ou la lectine qui reconnait une portion sucre produite est un anticorps ou une lectine marqué par une enzyme, et dans lequel on détermine le degré de développement de couleur formé par l'enzyme de marquage.

11. Procédé selon la revendication 8, dans lequel l'anticorps qui reconnaît une portion sucre produite est un anticorps non marqué, et dans lequel on détermine la quantité d'anticorps lié en utilisant un anticorps secondaire dirigé contre l'anticorps.

12. Procédé selon la revendication 8, dans lequel l'anticorps ou la lectine qui reconnait une portion sucre produite est un anticorps ou une lectine biotiné, et dans lequel on détermine la quantité de produit de réaction imobilisé en utilisant une enzyme avidinée.

13. Procédé de détermination des groupes sanguins qui comprend la détermination d'activités de glycosyltransférase dans un échantillon selon la technique en sandwich, par réaction de l'échantillon avec un système de réaction composé d'un donneur de sucre d'un nucléotide à sucre non marqué et d'un accepteur de sucre, qui est un oligosaccharide lié à une protéine non présente dans dans les fluides corporels humains, pour obtenir un produit de réaction, immobilisation du produit de réaction avec un anticorps insoluble qui reconnaît la portion protéique du produit de réaction, élimination de l'échantillon, par rinçage, d'un produit endogène de même structure que le produit de réaction, addition d'un anticorps ou d'une lectine qui reconnait une portion sucre produite par la réaction, et détermination de la présence de glycosyltransférase par mesure de la quantité de produit de réaction avec un anticorps ou une lectine qui réagit de façon spécifique avec le produit de réaction, ledit anticorps ou ladite lectine ne se fixant ni sur le donneur de sucre, ni sur l'accepteur de sucre sur lequel le sucre du donneur n'a pas encore été transféré.

14. Procédé de diagnostic du cancer, qui comprend la détermination d'activités de glycosyltransférase dans un échantillon selon une technique en sandwich, par réaction de l'échantillon avec un système de réaction composé d'un donneur de sucre d'un nucléotide à sucre non marqué et d'un accepteur de sucre, qui est un oligosaccharide lié à une protéine non présente dans dans les fluides corporels humains, pour obtenir un produit de réaction, immobilisation du produit de réaction avec un anticorps insoluble qui reconnait la portion protéique du produit de réaction, élimination de l'échantillon, par rinçage, d'un produit endogène de même structure que le produit de réaction, addition d'un anticorps ou d'une lectine qui reconnaît une portion sucre produite par la réaction et détermination de la présence de glycosyltransférase par mesure de la quantité de produit de réaction avec un anticorps ou une lectine qui réagit de façon spécifique avec le produit de réaction, ledit anticorps ou ladite lectine ne se fixant ni sur le donneur de sucre, ni sur l'accepteur de sucre sur lequel le sucre du donneur n'a pas encore été transféré.
